# EUROPEAN PATENT SPECIFICATION

(11) **EP 2 709 959 B1**
(45) Date of publication and mention of the grant of the patent: **04.01.2017**
(21) Application number: 12725482.9
(22) Date of filing: 16.05.2012
(51) Int. Cl.: C02F 1/461, C02F 1/467, A61L 2/03, C25B 1/13, C25B 9/04, C25B 11/02, C25B 11/04, A61L 2/18

(54) **ELECTRODE ASSEMBLY AND AN ELECTROCHEMICAL CELL COMPRISING THE SAME**
ELEKTRODENANORDNUNG UND BATTERIEZELLE DAMIT
ENSEMBLE ÉLECTRODE ET CELLULE ÉLECTROCHIMIQUE LE CONTENANT

(30) Priority: 17.05.2011 GB 201108253
(43) Date of publication of application: 26.03.2014
(73) Proprietor: A-Gas International, Portbury West Bristol BS20 7XH (GB)
(72) Inventor: BRAY, Patrick, St. Mawes Cornwall TR2 5AD (GB)
(74) Representative: Dean, Alexander Bruce
(86) International application number: PCT/GB2012/000436
(87) International publication number: WO 2012/156668

(56) References cited:
- EP-A1- 1 953 271
- WO-A2-02/061181
- DE-A1- 10 025 167
- JP-A- 2010 270 364
- US-A1- 2007 029 190
- US-A1- 2010 006 450
- US-A1- 2010 320 082

## Description

The present invention relates to an electrode assembly, in particular to an electrode assembly for use in an electrochemical cell, and to an electrochemical cell comprising the same. The present invention concerns in particular an electrode assembly and an electrochemical cell for the production of ozone and to a method of operating the cell.

Electrochemical cells find use in a range of applications for conducting a variety of electrochemical processes. In general, the cells comprise an anode and a cathode separated by a cation exchange membrane. One particular application for electrochemical cells is the production of ozone by the electrolysis of water.

Ozone is one of the strongest and fastest acting oxidants and disinfectants available for water treatment. Although ozone is only partially soluble in water, it is sufficiently soluble and stable to disinfect water contaminated by pathogenic micro-organisms and can be utilised for a wide range of disinfection applications including sterilisation. Micro-organisms of all types are destroyed by ozone and ozonated water including bacteria, viruses, fungi and fungal spores, oocysts, algae, and protozoa.

Ozone decomposes rapidly in water into oxygen and has a relatively short half life. The half life of ozone in water is dependant upon temperature, pH and other factors. As a result of its short half life, once treatment has been applied, ozone that remains in solution will rapidly decay to oxygen. Unlike chorine-based disinfectants, ozone does not form toxic halogenated intermediates and undesirable end products such as trihalomethanes (THMs).

The concentration of ozone dissolved in water determines the rate of oxidation and the degree of disinfection in any given volume of water; the higher the concentration of ozone, the faster the rate of disinfection of micro-organisms.

Electrolysis of water at high electrode potential produces ozone at the anode in an electrochemical cell according to the following equations:

3H₂O → O₃ + 6H⁺ + 6e⁻ (E₀ = 1.51 V_{SHE})

and

2H₂O → O₂ + 4H⁺ + 4e⁻ (E₀ = 1.23 V_{SHE})

H₂O + O₂ → O₃ + 2H⁺ + 2e⁻ (E₀ = 2.07 V_{SHE})

Ozone may be produced at higher current efficiencies and in higher concentrations from low conductivity water, deionised water, demineralised water, and softened water. The ozone may be produced in situ in the water stream by the above-mentioned anodic reactions as water flows through the electrochemical cell past the anode. Water with ozone dissolved therein is described as 'ozonated water'.

The production of ozone and ozonated water by electrolysis using an electrochemical cell is known in the art. DE 10025167 discloses an electrode assembly for use in a cell for the electrolytic production of ozone and/or oxygen. The cell comprises an anode and a cathode separated by a membrane in direct contact with each of the electrodes.

WO 2005/058761 discloses an electrolytic cell for the treatment of contaminated water. The cell comprises an anode and a cathode, with water being passed between the two electrodes. The cathode is preferably formed from nickel, titanium, graphite or a conductive metal oxide. The cathode is provided with a coating, preferably boron-doped diamond (BDD), activated carbon or graphite. The anode is preferably formed from titanium, niobium, or a conductive non-metallic material, such as p-doped silicon. The anode is preferably provided with a coating, with preferred coatings being boron doped diamond (BDD), lead oxide (PbO₂), tin oxide (SnO₂), platinised titanium, platinum, activated carbon and graphite.

US 2008/156642 concerns a system for the disinfection of low-conductivity liquids, in particular water, the system comprising an electrochemical cell in which electrodes are arranged to allow the liquid to flow therearound. Oxidizing agents, such as ozone, are produced from the liquid by the application of an electrical current.

US 2010/0320082 concerns a conductive diamond electrode and ozone generator employing the electrode. The electrode comprises a substrate having a plurality of convex and concave parts disposed over the entire surface of the electrode and coated with a film of diamond.

WO 02/061181 discloses a large dimension electrode having a metal support plate and a plurality of so-called tiles secured to a surface thereof. Each tile comprises a silicon-based substrate having a connecting layer in contact with the metal support plate and a conductive diamond layer.

US 2007/029190 concerns an electrode for electrolysis. The electrode comprises a substrate having a layer of conductive diamond material formed on its surface. The surface of the substrate may have a range of roughness.

JP 2005272910 discloses an electrode for electrolysis and comprising an electrically conducive diamond material. The diamond material is provided in the form of particles secured to one side of a metal plate.

A single crystal diamond electrochemical electrode is described and shown in US 2006/0261349.

US 2010/0006450 discloses a diamond electrode arrangement for use in an electrochemical cell for the treatment of water and/or the production of ozone. The cell comprises an anode and a cathode separated by a proton exchange membrane (PEM). The electrode is formed with a diamond plate and is configured to have one or more slots (described as elongated apertures) therein, to provide a minimum specified apertures length per unit of working area of the electrode. In particular, the electrode comprises an electrically conducting diamond plate comprising at least one aperture therein, the aperture having an edge length per unit working area of the diamond plate greater than about 4 mm/mm². It is described in US 2010/0006450 that when the electrode is used in the electrolysis of water to product ozone, the increase in edge length per unit working area of the diamond plate increases the rate of ozone production per unit working area of the electrode. The use of a diamond plate consisting essentially of diamond is preferred in US 2010/0006450, as this offers improved operational lifetimes of the electrode and also allows a wider variety of complex shaped diamond plates to be formed compared, for example, when a diamond-coated plate is used.

While the electrode assembly of US 2010/0006450 has been found to function well in the electrolysis of water to produce ozone, it has been found that its operational lifetime is relatively short. In particular, it has been found that the diamond plate is particularly fragile and is easily broken during the assembly or reassembly of the electrochemical cell or is fractured during operation of the cell. Once fractured or broken, the electrode cannot be repaired and must be replaced.

Accordingly, there is a need for an improved design of diamond electrode assembly.

In a first aspect, the present invention provides an electrode assembly for use in an electrochemical cell, the electrode assembly comprising a substrate comprising a plurality of substrate lands, each land having a working surface, the electrode assembly further comprising a layer of diamond on the working surface of the land, wherein the diamond layer is formed separately as a solid diamond material and is applied to the working surface of the land as a layer or sheet of solid diamond material; wherein adjacent lands are separated by a region of the substrate that is not provided with a layer of diamond.

Adjacent lands are separated by a region of the substrate that is not provided with a layer of diamond, in particular forming a conduit through which liquid may be brought into contact with the diamond material.

It has been found that the electrode assembly of the present invention is robust and has a long operating lifetime, capable of being assembled and disassembled easily without damage to the electrode. It has further been found that the electrode assembly is of particular advantage when used in an electrochemical cell for the electrolysis of water to produce ozone, being able to produce ozone at high rates and at a high current efficiency.

In a further aspect, the present invention provides an electrochemical cell for use in the electrolysis of water to produce ozone, the electrochemical cell comprising:
a first electrode assembly comprising an electrically conducting substrate comprising a plurality of substrate lands, each land having a working surface, the electrode assembly further comprising a layer of diamond on the working surface of the land, wherein the diamond layer is formed separately as a solid diamond material and is applied to the working surface of the land as a layer or sheet of solid diamond material; wherein adjacent lands are separated by a region of the substrate that is not provided with a layer of diamond; and a first fluid conduit for bringing fluid into contact with the layer of diamond of the first electrode;
a second electrode assembly and a second fluid conduit for bringing fluid into contact with the second electrode;
a cation exchange membrane extending between the first electrode assembly and the second electrode assembly and separating the first fluid conduit from the second fluid conduit; and
an electrical supply system for providing an electric current between the first and second electrode assemblies.

The electrode assembly of the present invention comprises a substrate. The substrate may be any suitable electrically conductive material, with the diamond material being in electrical contact with the substrate, for example by direct contact between the diamond and the substrate material or by use of a suitable conductive medium, such as an electrically conducting adhesive. Suitable materials for use as the electrode substrate include metals, such as tungsten, niobium, molybdenum, platinum, palladium, titanium and tantalum. A particularly preferred metal is titanium. Other materials for use as the substrate include semiconductor materials, such as doped silicon, for example p-doped silicon. Suitable materials for use as the substrate are known in the art and are commercially available.

However, it has been found that, depending upon the application of the electrochemical cell, the conditions prevailing at the diamond electrode during use can erode the electrode substrate. This is particularly the case when the electrochemical cell is being used for the production of ozone by the electrolysis of water.

Alternatively, the electrode substrate is formed from a material that is not electrically conductive. Suitable non-conductive materials include plastics and resins. Thermoplastic fluoropolymers are a preferred class of materials for forming the electrode substrate, in particular polyvinylidene fluoride (PVDF). Another suitable material is polymethylmethacrylate (PMMA) known as acrylic. In embodiments using a non-conductive substrate material, it is necessary to provide means for providing an electrical current to the diamond electrodes. This may be achieved using any suitable arrangement of wires or current feeders. In one preferred arrangement, the electrode substrate is formed with one or more current feeders extending therethrough to provide current to the one or more diamond electrodes. The current feeders are conveniently connected to a single electrical supply, for example by use of a bus bar or the like.

As noted above, due to the corrosive nature of the conditions prevailing at the diamond electrodes during use of the cell, it is preferred that means are provided to prevent the current feeders or wires supplying electrical current to the diamond electrodes from being contacted by the anolyte or the catholyte. Suitable means include a layer of suitably inert material to separate the current feeders and/or the wires from the electrolyte solutions. In one preferred embodiment, the diamond electrodes and means for providing current thereto are sealed within a non-conducting substrate material, for example by having the substrate material cast therearound.

The electrode substrate is formed with a plurality of lands spaced apart from one another. The electrode substrate may have any suitable form to provide the lands having a working surface. The electrode substrate may be formed from a plurality of substrate components, each providing one or more lands. In this arrangement, the separate components are arranged to be connected to a source of electrical current, for example by suitable electrical connections, such as current feeders or wires.

In one embodiment, the electrode substrate is formed as an electrode body, the body having lands formed as raised portions on a region of its surface. Each raised portion may have any suitable form and cross-section so as to provide a working surface for the electrode substrate. Each raised portion is preferably rectangular in cross-section.

In one preferred embodiment, the electrode substrate comprises a plate-like body, having a plurality of grooves formed in a major surface thereof, adjacent grooves defining a land therebetween. The grooves and lands are preferably rectangular in cross-section and may be formed in the electrode body by known techniques, such as machining. The grooves in the electrode body are not provided with a diamond material and provide a conduit through which liquid may be passed, to contact the adjacent surfaces of the electrode, as described in more detail hereinafter.

The lands of the electrode substrate are preferably elongate, that is having a length significantly greater than their width, preferably a length that is from 2 to 30, more preferably from 5 to 20, more preferably from 6 to 10 times the width. The lands may have any suitable width. This will be determined by the desired or required dimensions of the diamond material on the working surface of the electrode substrate, as described in more detail below.

As noted above, the electrode assembly comprises a plurality of lands, each land provided with a layer of diamond on its working surface. Each land is provided with its own layer of diamond, separate from the layer of diamond present on other lands that may be present in the cell. In this way, the layer of diamond on each land is able to adapt to conditions local to it in the cell, without affecting the state or performance of the diamond material on the other lands in the cell. This in turn leads to a significantly more robust cell structure, with a longer operating lifetime and greater operating efficiency.

The layer of diamond may comprise any suitable electrically conductive diamond materials. Such diamond materials are known in the art and are commercially available. The electrically conductive diamond material may be a layer of single crystal synthetic diamond or polycrystalline diamond. Polycrystalline diamond is particularly preferred. The electrically conductive diamond may be synthesised using techniques known in the art, for example by way of high pressure high temperature (HPHT) or by way of chemical vapour deposition (CVD), with CVD diamond again being preferred. The diamond consists essentially of carbon matrix doped with one or more elements. Diamond doped with boron, described as boron-doped diamond (BDD), is preferred. A particularly preferred diamond material is polycrystalline boron-doped diamond.

The diamond material is provided separately and mounted on the working surface of the land of the electrode substrate. The diamond material may be applied to the working surface of the land of the electrode substrate by any suitable means. For example, the diamond material may be mounted on the working surface by an adhesive, for example an epoxy adhesive. Suitable adhesives are known in the art and are commercially available. The selection of the adhesive may be determined by the use to be made of the electrode assembly and the electrochemical cell in which it is installed. In particular, the adhesive should be resistant to the conditions prevailing within the cell when in operation. In the electrolysis of water, the conditions within the electrochemical cell may be harsh, with a high concentration of oxidants and acids at the electrodes. Accordingly, any adhesive used to secure the diamond material to the working surface of the land of the substrate is preferably resistant to such conditions.

In one embodiment of electrochemical cell, described in more detail below, the first and second electrode assemblies are arranged on opposing sides of a cation exchange membrane, with the surface of the diamond layer of each electrode in contact with the electrode. In such an arrangement, in which the diamond material is in contact with another component within the cell, such as the membrane, it has been found that it is not necessary to provide means, such as an adhesive, to secure the diamond material to the working surface of the land of the electrode substrate. Rather, in one embodiment employing an electrically conducting substrate material, the electrochemical cell may be assembled with the diamond material as a sheet held between the working surface of the land of the substrate and the adjacent component, in particular the membrane. An electrically conductive adhesive may be applied between the sheet of diamond material and the working surface of the substrate to aid in assembly. Suitable electrically conducting adhesives are known in the art and are commercially available.

More preferably, the substrate material is non-conducting. In this arrangement, the diamond electrode is provided on a land on the electrode substrate and connected to means for providing electrical current, in particular a current feeder extending through the electrode substrate as described hereinbefore. In a particularly preferred arrangement, the diamond electrode is connected to the current feeder by a suitable electrically conducting adhesive, with silver epoxy cement being particularly preferred. Each diamond electrode is then secured to the substrate by having the substrate material applied or cast around the diamond electrode and current feeder assembly. In this way, the diamond electrode is retained in position within the cell.

As noted above, it has been found that problems may arise in the electrochemical cell during operation due to conditions prevailing at the electrode eroding components, such as the adhesive or cement used to mount the diamond electrode to the electrode substrate and the current feeders providing electrical current to the diamond electrodes. It has been found that casting the electrode substrate around the components, in particular the diamond electrode and current feeders in a suitable mould allows the vulnerable components to be sealed against such erosion.

The diamond material on the working surface of the land of the electrode substrate may have any suitable dimensions. The thickness of the diamond material will depend upon its manner of preparation.

The diamond is formed separately as a solid diamond material and applied to the working surface of the electrode substrate as a layer or sheet of solid diamond material. It has been found that a problem occurs with a diamond layer on the substrate that is formed by coating the substrate. In particular, it has been found that the edges of the diamond material erode under the action of the cell, eventually leading to failure of the diamond electrode and the cell. It has been found that forming a solid diamond material and then applying the diamond material as a layer to the substrate prevents the edges of the diamond material from being eroded under the action of the cell and significantly increases the longevity and working life of the cell. In this case, the diamond material has a thickness of from 100 to 2000 microns, more preferably from 200 to 1000 microns, still more preferably from 300 to 800 microns. A thickness of from 400 to 700 microns, in particular about 600 microns, is especially preferred.

The diamond material is a sheet of material on the land of the electrode substrate, as described hereinbefore. In this arrangement, the diamond material may have any suitable length and width. It is preferred that the width of the diamond material is large enough to protect the underlying substrate material and prevents its degradation and erosion. The diamond material is preferably of a width of from 2 mm to 20 mm, more preferably from 2 mm to 10 mm, still more preferably from 4 to 8 mm. A width of diamond material in this range has been found to prevent the erosion and degradation of the underlying substrate material. The length of the diamond material may be in the range of from 10 mm to 100 mm, more preferably from 10 mm to 50 mm, still more preferably from 10 mm to 20 mm.

In use, the electrode assembly is connected to a source of electrical current, in particular to connect the diamond material on each land of the substrate to a source of electrical current. In one embodiment, the electrode substrate is electrically conductive and the diamond material is in electrical contact with the substrate. Accordingly, the electrode assembly is provided with a suitable connection for connecting to an electrical current. Alternatively, the electrode substrate is electrically non-conducting, in which case the electrode assembly is provided with means for supplying electrical current to the diamond electrode, such as one or more current feeders. Such current feeders are in turn connected to a suitable connection means for further connection to an electrical supply. Any suitable connection means may be employed, such as terminals, for example terminals threaded into the substrate.

The electrode assembly of the present invention finds use in electrochemical cells, in particular where an electrode comprising a diamond material is required for the electrochemical reactions. As noted above, the electrode assembly finds particular use in an electrochemical cell for the electrolysis of water, especially for the production of ozone and/or ozonated water.

A preferred electrochemical cell comprises a first electrode assembly of the present invention and a second electrode assembly of the present invention on opposing sides of a cation exchange membrane. The membrane permits the movement of cations, including hydrogen ions (protons) and positively charged metal cations, such as calcium and magnesium, in the anolyte to pass through the membrane to the cathode and catholyte, in either direction, depending upon the polarity of the current applied to the cell at any given time.

The membrane is in contact with each electrode. Each electrode assembly is provided with a conduit for providing a fluid to contact the respective electrode, in particular the diamond material, the two conduits being separated by the membrane. In the preferred embodiment described above, in which the electrode substrate comprises a plurality of lands formed therein, with adjacent lands separated by grooves or channels in the substrate, the grooves or channels are not provided with a diamond material and provide the conduit for the passage of fluid to the active portions of the electrode.

Each electrode assembly is formed to have edges to the active surface of the diamond material, with the cation exchange membrane being in contact with the edges of the diamond material. In this way, electrochemical reactions take place at the interface between the anode, the membrane and the liquid in the conduit adjacent the anode. For example, in the case of the electrolysis of water, ozone is produced in the water stream in the conduit at the junction of the diamond material and the membrane, most particularly at the edge of the diamond material in contact with the membrane to produce water having a significant concentration of ozone (ozonated water). Hydrogen ions (protons) pass through the membrane to the cathode side of the cell where hydrogen gas is produced. Other positively charged metal cations such as calcium, magnesium, iron and manganese also pass through the membrane and are deposited on the cathode.

Suitable materials for the membrane are known in the art and are commercially available. One particularly preferred class of materials for use in the membrane are sulfonated tetrafluoroethylene-based fluoropolymers. Such materials are known in the art and are commercially available, for example the Nafion® range of products available from Dow Chemical.

During operation of the electrochemical cell, liquid, for example water, is supplied to the conduits on the side of the membrane adjacent the first electrode acting as the anode and forms the anolyte. Liquid, for example water, is also supplied to the conduits on the side of the membrane adjacent the second electrode acting as the cathode and forms the catholyte. In a preferred embodiment, the electrochemical cell is operated under a regime of forced flow circulation. Liquid is supplied to the cell under pressure and is evenly distributed either side of the membrane by means of inlet manifolds connected to the conduits adjacent both the first and second electrodes. The electrochemical reactions that take place when the cell is operating are therefore uniformly distributed in the conduits adjacent to the edges of the electrodes.

In one embodiment, the liquid supplied to the electrochemical cell is divided equally between the anolyte and catholyte sides of the cell. In this way, the liquid pressure either side of the membrane in the cell is equal, regardless of the rate of flow of liquid through the cell. Therefore, there is no hydrostatic force driving anolyte through the membrane into the catholyte side of the cell. Further, as the liquid supply is common to both sides of the membrane, the concentration of the anolyte and catholyte may also be maintained substantially equal. Therefore, there is no osmotic force present to drive liquid, such as water, from the anolyte side of the cell into the catholyte side of the membrane.

The cell may be provided with liquid at any suitable flow rate. The liquid flow through the electrochemical cell is determined several factors including the size of the cell, the number of flow channels through the cell and the dimensions of the flow channels. The electrochemical reactions occurring within the cell and therefore cell performance increases as the rate of mass transport increases at the interface of the anode, the membrane and the liquid flowing through the cell. Increasing the rate of flow of liquid to the optimum increases mass transport within the cell, and therefore cell efficiency. The range of flow of liquid through the cell is between 0.5 and 50 litres/minute depending upon the size of the electrochemical cell and the factors described above.

The electrochemical cell can be operated at a wide range of liquid pressures, for example, in the case of the electrolysis of water, the cell may be operated most conveniently at mains water pressure. Liquid pressures of at least 1 Bar, more preferably at least 2 Bar, may be employed, with the maximum liquid pressure preferably being 10 Bar, more preferably 8 Bar. To obtain the optimum efficiency of the electrochemical cell the pressure should be regulated to maintain the optimum flow as described above.

The electrochemical cell is provided with a supply of electrical current to the electrodes. The operating current density, measured in Amps/m², at the electrodes is a function of the electrical current applied to the cell, measured in amps, from the electrical power supply, divided by the active surface area of the diamond electrodes. The current applied to the electrochemical cell, and therefore the current density at the electrodes, may be selected to optimise the performance of the cell, for example to optimise the production of ozone and ozonated water.

The electrochemical cell may be operated at any suitable current densities, with current densities up to 5.0 Amps/cm² being preferred, depending upon the size and duty of the cell. Preferably, the current density is in the range 0.1 to 5.0 Amps/cm², and more preferably in the range 0.2 to 3.4 Amps/cm², still more preferably from 0.4 to 1.7 Amps/cm², for the production of ozonated water for most disinfection applications.

The electrical current applied to the electrochemical cell is determined by the surface area of the active anodes in the cell and the current density to generate the required concentration of ozone and ozone water. The minimum electrical current applied to the smallest cell is preferably 0.1 Amp, more preferably 0.5 Amp, and the maximum current applied to a largest cell may be up to 20 Amps.

The electrochemical cell may be operated at any suitable applied voltage, with voltages up to 50 Volts, more preferably up to 40 Volts being preferred, depending upon the conductivity of the liquid stream being treated. According to the operating conditions, the cell voltage is preferably from 5 Volts to 30 Volts, more preferably from 12 Volts to 24 Volts.

Embodiments of the present invention will now be described, by way of example only, having reference to the accompanying drawings, in which:
Figure 1 is a perspective view of an electrode assembly according to one embodiment of the present invention;
Figure 2 is an end view of the electrode assembly of Figure 1 along the line II - II;
Figure 3 is a diagrammatical cross-sectional view of an electrochemical cell of one embodiment of the present invention comprising electrode assemblies as shown in Figure 1;
Figure 4 is a diagrammatical representation of a system for the production of ozone and/or ozonated water by the electrolysis of water; and
Figure 5 is a diagrammatical cross-sectional view of an electrochemical cell of a further embodiment of the present invention.

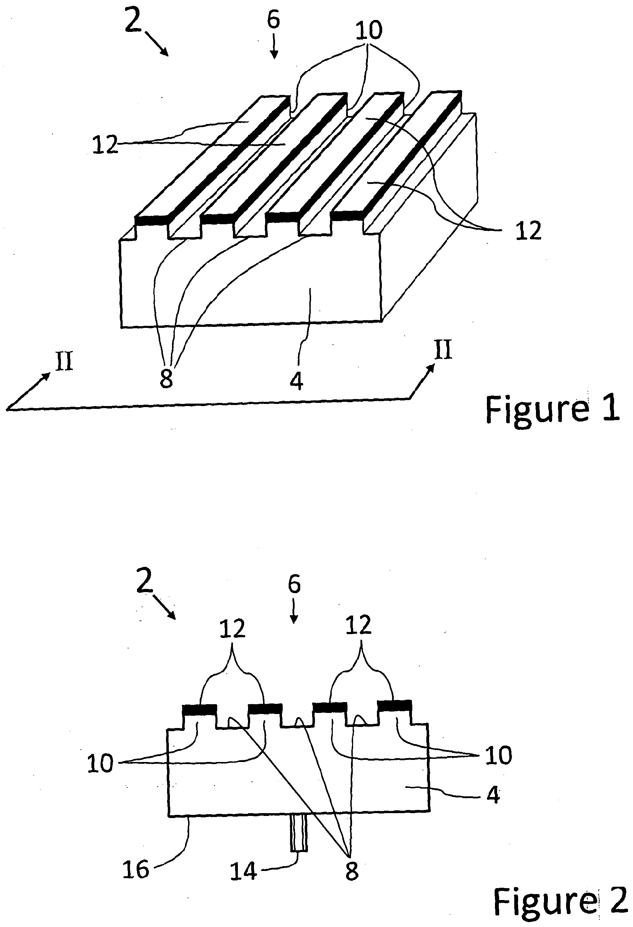

Referring to Figure 1, there is shown one embodiment of the electrode assembly of the present invention, generally indicated as 2. The electrode assembly comprises an electrode substrate having a generally rectangular substrate body 4 of generally rectangular cross-section. The substrate body 4 has a major surface 6, in which are formed a plurality of channels 8, defining therebetween a plurality of lands 10. In use, as described below, the channels 8 provide a conduit for the passage of liquid, such as water, to be electrolysed. The channels 8 may be formed by any suitable means, for example by machining, in the major surface 6 of the substrate body 4. The channels 8 and the lands 10 each have a generally rectangular cross-section.

As shown in Figure 1, the substrate body is formed with 4 lands. However, it is to be understood that other numbers of lands may be employed, depending upon the size and use of the electrochemical cell.

The substrate body may be formed from any electrically conductive material, in particular titanium.

Each land 10 has a working surface, to which is applied a pre-formed sheet 12 of solid diamond material, in particular boron doped diamond (BDD). Each sheet 12 of diamond material is generally rectangular in cross-section. The sheet of diamond material may be secured to the working surface of the respective land by any suitable means, for example by being adhered thereto. Preferably, the diamond material is applied to each land using an electrically conducting grease, to retain the sheet 12 of the diamond material in place while the electrode assembly is assembled into an electrochemical cell. Each sheet 12 of diamond material shown in Figure 1 has a thickness of about 300 to 600 microns.

Referring to Figure 2, the electrode assembly 2 of Figure 1 is shown in end view. The electrode assembly 2 is provided with a stud 14 threaded into a blind bore in the major face 16 of the substrate body opposing the lands and channels. The stud 14 provides a means for mounting the electrode assembly in a suitable electrochemical cell and provides a means for connecting the electrode assembly to a suitable supply of electrical current.

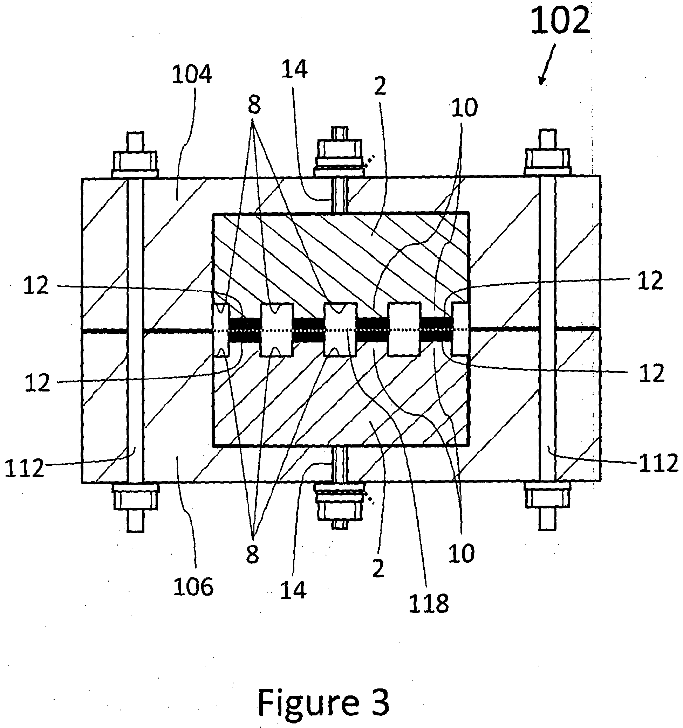

Turning to Figure 3, there is shown a cross-sectional view of an electrochemical cell, generally indicated as 102, for the production of ozone and ozonated water by the electrolysis of water. The cell 102 comprises a pair of opposing cell bodies 104, 106 held together by means of through bolts 112. The first and second cell bodies 104, 106 may be prepared from any suitable material that is chemically resistant to strong oxidants, in particular ozone, generated within the cell. Thermoplastic fluoropolymers are a preferred class of materials for forming the cell bodies, in particular polyvinylidene fluoride (PVDF). Another suitable material is polymethylmethacrylate (PMMA), also known as acrylic. The bolts clamping the first and second cell bodies 104, 106 together may be of any suitable material with sufficient strength to hold the cell bodies together, preferably corrosion resistant metals, such as stainless steel.

Each cell body 104, 106 is provided with an electrode assembly 2, as shown in Figures 1 and 2 and described above. Each electrode assembly 2 is retained within a cavity formed within the respective cell body 104, 106. The electrode assemblies 2 are arranged to have the channels 8 and lands 10 in the major face of the cell bodies oppose each other, such that the channels in the cell bodies are arranged in opposing pairs. Each channel 8 is not provided with a layer of diamond and forms a conduit for the passage of water therethrough for contacting the active diamond material on each land 10. Similarly, the lands in the major face of each electrode assembly and the BDD sheets 12 are arranged to form opposing pairs.

A cation exchange membrane 118 extends between the first and second cell bodies 104, 106. The membrane 118 is formed from a sulfonated tetrafluoroethylene-based fluoropolymer (Nafion^{®} N-117 membrane). The membrane 118 divides the opposing channels 8 in the faces of the electrode assemblies. Further, the membrane 118 extends between the opposing pairs of BDD sheets 12. The electrode assemblies 2, the BDD electrodes 12 and the membrane 18 are arranged such that the surface of each of the BDD electrodes is in contact with the adjacent portion of the membrane.

Each electrode assembly 2 is secured in the respective cell body 104, 106 by means of the stud 14 and a nut. The stud extends from the outer surface of the respective cell body 104, 106 and provides means for connecting the electrode assembly to a source of electrical current.

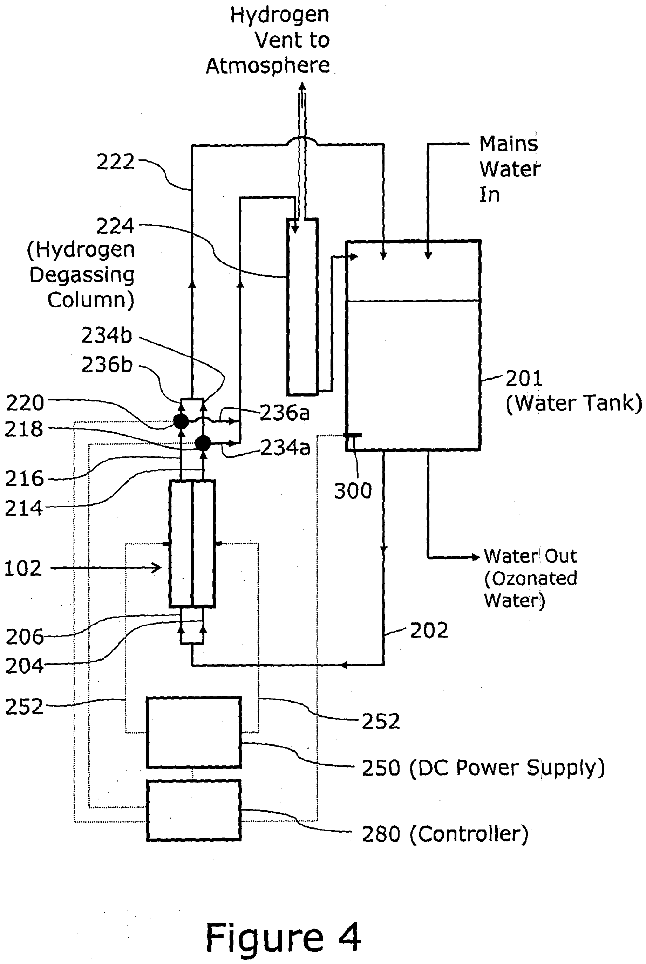

In use for the production of ozone by the electrolysis of water, the electrochemical cell 102 shown in Figure 3 is connected to a system as shown in Figure 4. Referring to Figure 4, the electrochemical cell 102 is provided with a supply of fresh water, for example mains water. The water is supplied to the cell through a line 202, via a tank 201, and divided equally between lines 204 and 206, each feeding water from the supply to the channels 8 in respective electrode assemblies 2. In this way, the channels and electrodes on each side of the membrane are provided with water having the same composition and under the same conditions of pressure and flowrate.

Liquid leaves the channels 8 in the electrode assemblies 2 of the cell bodies 104, 106 through respective lines 214, 216, each provided with a solenoid valve 218, 220. Each solenoid valve 218, 220 is operable to direct the liquid leaving the cell 102 to either an ozonated water product line 222 or a hydrogen degassing column 224. Thus, liquid may be directed from the solenoid valve 218 along either a line 234a to the hydrogen degassing column 224 or a line 234b to the product line 222. Similarly, liquid may be directed from the solenoid valve 220 along a line 236a to the hydrogen degassing column 224 or a line 236b to the product line 222.

Hydrogen gas is effectively removed from the catholyte water stream by the hydrogen degassing column 224 and safely vented to atmosphere. After degassing the water is returned to the process tank 201 as shown on Figure 4.

A supply of direct current (DC current) is provided to the electrochemical cell 102 by a DC power supply system 250, connected by cables 252 to the studs 14 of each electrode assembly. The electric supply system 250 is operable to provide current to the electrodes as required and with either of two polarities.

In operation, the electrochemical cell is operated with the water supplied to the channels 8 in both electrode assemblies 2. Current is supplied to the cell by the electric supply system 250 with a first polarity, such that the BDD electrodes in the first electrode assembly 2 act as the anode and the BDD electrodes in the second electrode assembly s act as the cathode. Anolyte produced in the channels of the first electrode assembly leaves through the line 214 and is directed by the solenoid valve 218 to the line 234b to the product outlet line 222. Catholyte produced in the channels of the second electrode assembly leaves through the line 216 and is directed by the solenoid valve 220 to the line 236a, to be fed to the hydrogen degasser 224.

By changing the position of the solenoid valves 218, 220 and reversing the polarity of the electrical supply, the operation of the cell may be reversed, whereby the first electrode assembly 2 acts as the cathode and the second electrode assembly 2 acts as the anode.

Operation of the system is controlled by means of a controller 280, in particular allowing the position of the solenoid valves 218, 220 to be changed and the DC power supply 250 to be controlled, in particular to change the current polarity. The controller 280 further controls the concentration of ozone in the water in the tank 201, by means of an oxidation/reduction potential (ORP) sensor or ozone sensor 300 in the base of the tank 201. In operation, the controller 280 determines the concentration of ozone in the water in the tank 201 by means of the signal received from the sensor 300. The operation of the electrochemical cell is controlled to maintain the ozone concentration in the tank within the desired range, determined by the end use to be made of the ozonated water.

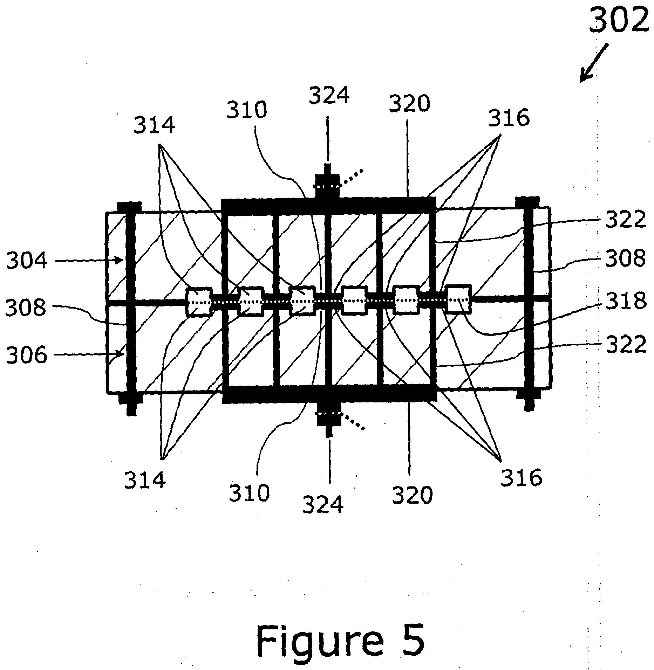

Turning to Figure 5, there is shown an electrochemical cell, generally indicated as 302, for the production of ozone and/or ozonated water by the electrolysis of water. The cell 302 comprises a pair of opposing cell bodies 304, 306 held together by means of a number of through bolts 308. The first and second cell bodies 304, 306 may be prepared from any suitable material that is chemically resistant to strong oxidants, in particular ozone, generated within the cell. Thermoplastic fluoropolymers are a preferred class of materials for forming the cell bodies, in particular polyvinylidene fluoride (PVDF). Another suitable material is polymethylmethacrylate (PMMA) known as acrylic. Another preferred material is a liquid resin system, such as catalysed epoxy resin, that is poured into a cell mould and sets around the components of the cell forming a solid cell body, when cured, that effectively seals the components within the cell.

Each cell body 304, 306 is provided with a plurality of elongate cavities in a major face, with each adjacent pair of cavities as shown in Figure 5 being separated by a land 310. The cavities and lands are arranged in the face of the cell bodies 304, 306 such that the cavities in the cell bodies are arranged in opposing pairs. Each cavity forms a conduit 314 for the passage of water therethrough. As shown in Figure 5, the conduits 314 have a generally rectangular cross-section. Similarly, the lands 310 in the face of each cell body 304, 306 are arranged to form opposing pairs. The surface of each land is provided with a layer of boron doped diamond (BDD) 316. The layers of BDD on the first cell body 304 form a plurality of first electrodes, while the layers of BDD on the second cell body 306 form a plurality of second electrodes.

A cation exchange membrane 318 extends between the first and second cell bodies 304, 306. The membrane 318 is formed from a sulfonated tetrafluoroethylene-based fluoropolymer (Nafion® N-117 membrane). The membrane 318 divides the opposing conduits 314 in the faces of the cell bodies. Further, the membrane 318 extends between the opposing pairs of BDD electrodes. The cell bodies 304, 306, the BDD electrodes 316 and the membrane 318 are arranged such that the surface of each of the BDD electrodes is in contact with the adjacent portion of the membrane.

Each cell body 304, 306 of the cell 302 is provided with a busbar 320, from which extend current feeders 322 connecting the busbar 320 with the respective BDD electrodes 316. The busbar 320 and current feeders 322 may be formed from any suitable conductor, for example stainless steel, aluminium, copper or brass. Copper is particularly preferred. Each diamond electrode 316 is connected to the respective current feeder 322 by silver epoxy cement.

Each busbar 320 is further provided with a current connector 324, allowing the busbar to be connected to an electrical supply system.

The current feeders 322 and busbars 320 are sealed within the cell bodies 304, 306 to prevent leakage from the conduits 314. In addition, the cell bodies 304, 306 are arranged to prevent electrolyte in the conduits 314 from reaching the current feeders. In particular, each cell body 304, 306 is cast around the diamond electrodes 316 and the current feeders 322, so as to encapsulate them and the silver epoxy cement, leaving the surface and edges of the BDD electrodes exposed. In this way, liquid electrolyte in the conduits 314 is prevented from having contact with the cement and current feeders, thereby reducing or preventing erosion.

The electrochemical cell 302 shown in Figure 5 is operated in like manner to the cell of Figure 1, for example in a system for producing ozonated water as described above and shown in Figure 4.

## Claims

1. An electrode assembly for use in an electrochemical cell, the electrode assembly comprising a substrate comprising a plurality of substrate lands, each land having a working surface, the electrode assembly further comprising a layer of diamond on the working surface of the land, wherein the diamond layer is formed separately as a solid diamond material and is applied to the working surface of the land as a layer or sheet of solid diamond material; wherein adjacent lands are separated by a region of the substrate that is not provided with a layer of diamond.

2. The electrode assembly according to claim 1, wherein the substrate is electrically conducting, preferably wherein the substrate comprises tungsten, niobium, molybdenum, platinum, palladium, titanium, tantalum or doped silicon.

3. The electrode assembly according to claim 1, wherein the electrode substrate is formed from an electrically non-conducting material, preferably wherein the substrate material comprises a fluoropolymer or an acrylic resin.

4. The electrode assembly according to any preceding claim, wherein the electrode substrate is provided with one or more lands formed as raised portions on a region of the surface of the electrode substrate.

5. The electrode assembly according to any preceding claim, wherein the electrode substrate is provided with a plurality of grooves extending in a surface thereof, adjacent grooves defining a land therebetween.

6. The electrode assembly according to any preceding claim, wherein the diamond layer comprises polycrystalline diamond.

7. The electrode assembly according to any preceding claim, wherein the diamond of the diamond layer is doped, preferably wherein the diamond is boron doped diamond (BDD).

8. The electrode assembly according to any preceding claim, wherein the diamond layer is applied to the working surface using an adhesive.

9. The electrode assembly according to any preceding claim, wherein the diamond layer has a thickness of from 100 to 2000 microns.

10. The electrode assembly according to any preceding claim, wherein the diamond layer has a width of from 2 to 20 mm.

11. The electrode assembly according to any preceding claim, wherein the diamond layer has a length of from 10 to 100 mm.

12. An electrochemical cell comprising:
a first electrode assembly as claimed in any preceding claim and a first fluid conduit for bringing fluid into contact with the layer of diamond of the first electrode;
a second electrode assembly and a second fluid conduit for bringing fluid into contact with the second electrode;
a cation exchange membrane extending between the first electrode assembly and the second electrode assembly and separating the first fluid conduit from the second fluid conduit; and
an electrical supply system for providing an electric current between the first and second electrode assemblies.

13. The electrochemical cell according to claim 12, wherein the second electrode assembly is as claimed in any of claims 1 to 11.

14. The electrochemical cell according to either of claims 12 or 13, wherein the cation exchange membrane comprises a sulfonated tetrafluoroethylene-based fluoropolymer.

15. The use of an electrode assembly according to any of claims 1 to 11 or an electrochemical cell according to any of claims 12 to 14 in the electrolysis of a liquid, preferably wherein the electrolysis is of water to produce ozone.

## Patentansprüche

1. Elektrodenanordnung zur Verwendung in einer elektrochemischen Zelle, die Elektrodenanordnung umfassend ein Substrat, welches eine Mehrzahl von Substrat-Stegen umfasst, wobei jeder Steg eine Arbeitsfläche aufweist, die Elektrodenanordnung des Weiteren umfassend eine Schicht aus Diamant auf der Arbeitsfläche des Stegs, wobei die Diamantschicht separat als ein massives Diamantmaterial gebildet ist und an der Arbeitsfläche des Stegs als eine Schicht oder Platte aus solidem Diamantmaterial befestigt ist; wobei benachbarte Stege durch einen Bereich des Substrates, der nicht mit der Diamantschicht ausgestattet ist, voneinander getrennt sind.

2. Elektrodenanordnung nach Anspruch 1, wobei das Substrat elektrisch leitend ist, vorzugsweise wobei das Substrat Wolfram, Niob, Molybdän, Platin, Palladium, Titan, Tantal oder dotiertes Silizium umfasst.

3. Elektrodenanordnung nach Anspruch 1, wobei das Elektrodensubstrat aus einem nicht elektrisch leitenden Material gebildet ist, vorzugsweise wobei das Substratmaterial ein Fluorpolymer oder ein Acrylharz umfasst.

4. Elektrodenanordnung nach einem der vorhergehenden Ansprüche, wobei das Elektrodensubstrat mit einem oder mehreren Stegen als erhöhte Abschnitte auf einem Bereich auf der Oberfläche des Elektrodensubstrats ausgestattet ist.

5. Elektrodenanordnung nach einem der vorhergehenden Ansprüche, wobei das Elektrodensubstrat mit einer Mehrzahl von über die Oberfläche hiervon verlaufenden Nuten ausgestattet ist, wobei benachbarte Nuten dazwischen einen Steg definieren.

6. Elektrodenanordnung nach einem der vorhergehenden Ansprüche, wobei die Diamantschicht polykristallinen Diamant umfasst.

7. Elektrodenanordnung nach einem der vorhergehenden Ansprüche, wobei der Diamant der Diamantschicht dotiert ist, vorzugsweise wobei der Diamant Bor-dotierter Diamant (BDD) ist.

8. Elektrodenanordnung nach einem der vorhergehenden Ansprüche, wobei die Diamantschicht unter Einsatz eines Klebers auf die Arbeitsfläche aufgebracht ist.

9. Elektrodenanordnung nach einem der vorhergehenden Ansprüche, wobei die Diamantschicht eine Dicke von 100 bis 2000 Mikrometern aufweist.

10. Elektrodenanordnung nach einem der vorhergehenden Ansprüche, wobei die Diamantschicht eine Breite von 2 bis 20 mm aufweist.

11. Elektrodenanordnung nach einem der vorhergehenden Ansprüche, wobei die Diamantschicht eine Länge von 10 bis 100 mm aufweist.

12. Eine elektrochemische Zelle, umfassend:
eine erste Elektrodenanordnung nach einem der vorhergehenden Ansprüche und eine erste Fluidleitung, um Fluid in Kontakt mit der Schicht aus Diamant der ersten Elektrode zu bringen;
eine zweite Elektrodenanordnung und eine zweite Fluidleitung, um Fluid in Kontakt mit der zweiten Elektrode zu bringen:
eine Kationenaustauschermembran, die sich zwischen der ersten Elektrodenanordnung und der zweiten Elektrodenanordnung erstreckt und die erste Fluidleitung von der zweiten Fluidleitung trennt; und
ein elektrisches Versorgungssystem, um einen elektrischen Strom zwischen der ersten und zweiten Elektrodenanordnung bereitzustellen.

13. Elektrochemische Zelle nach Anspruch 12, wobei die zweite Elektrodenanordnung ist, wie in einem der Ansprüche 1 bis 11 beansprucht.

14. Elektrochemische Zelle nach einem der Ansprüche 12 oder 13, wobei die Kationenaustauschermembran ein Fluorpolymer umfasst, welches auf sulfoniertes Tetrafluorethylen basiert.

15. Verwendung einer Elektrodenanordnung nach einem der Ansprüche 1 bis 11 oder einer elektrochemischen Zelle nach einem der Ansprüche 12 bis 14 in der Elektrolyse einer Flüssigkeit, wobei es vorzugsweise die Elektrolyse von Wasser zur Ozonproduktion ist.

## Revendications

1. Ensemble d'électrodes pour une utilisation dans une cellule électrochimique, l'ensemble d'électrodes comprenant un substrat comprenant une pluralité de pastilles de substrat, chaque pastille ayant une surface de travail, l'ensemble d'électrodes comprenant en outre une couche de diamant sur la surface de travail de la pastille, dans lequel la couche de diamant est formée séparément comme un diamant solide et est appliquée à la surface de travail de la pastille comme une couche ou une feuille de diamant solide ; dans lequel des pastilles adjacentes sont séparées par une zone du substrat qui n'est pas pourvue d'une couche de diamant.

2. Ensemble d'électrodes selon la revendication 1, dans lequel le substrat est électriquement conducteur, de préférence dans lequel le substrat comprend du tungstène, du niobium, du molybdène, du platine, du palladium, du titane, du tantale ou du silicium dopé.

3. Ensemble d'électrodes selon la revendication 1, dans lequel le substrat d'électrode est formé à partir d'un matériau électriquement non conducteur, de préférence dans lequel le matériau de substrat comprend un fluoropolymère ou une résine acrylique.

4. Ensemble d'électrodes selon l'une quelconque des revendications précédentes, dans lequel le substrat d'électrode est pourvu d'une ou plusieurs pastilles formées comme des parties surélevées sur une zone de la surface du substrat d'électrode.

5. Ensemble d'électrodes selon l'une quelconque des revendications précédentes, dans lequel le substrat d'électrode est pourvu d'une pluralité de rainures s'étendant dans une surface de celui-ci, des rainures adjacentes définissant une pastille entre celles-ci.

6. Ensemble d'électrodes selon l'une quelconque des revendications précédentes, dans lequel la couche de diamant comprend un diamant polycristallin.

7. Ensemble d'électrodes selon l'une quelconque des revendications précédentes, dans lequel le diamant de la couche de diamant est dopé, de préférence dans lequel le diamant est un diamant dopé au bore (BDD).

8. Ensemble d'électrodes selon l'une quelconque des revendications précédentes, dans lequel la couche de diamant est appliquée à la surface de travail en utilisant un adhésif.

9. Ensemble d'électrodes selon l'une quelconque des revendications précédentes, dans lequel la couche de diamant a une épaisseur de 100 à 2 000 microns.

10. Ensemble d'électrodes selon l'une quelconque des revendications précédentes, dans lequel la couche de diamant a une largeur de 2 à 20 mm.

11. Ensemble d'électrodes selon l'une quelconque des revendications précédentes, dans lequel la couche de diamant a une longueur de 10 à 100 mm.

12. Cellule électrochimique comprenant :
un premier ensemble d'électrodes selon l'une quelconque des revendications précédentes et une première conduite de fluide pour mettre du fluide en contact avec la couche de diamant de la première électrode ;
un second ensemble d'électrodes et une seconde conduite de fluide pour mettre du fluide en contact avec la seconde électrode ;
une membrane échangeuse de cations s'étendant entre le premier ensemble d'électrodes et le second ensemble d'électrodes et séparant la première conduite de fluide de la seconde conduite de fluide ; et
un système d'alimentation électrique pour fournir un courant électrique entre les premier et second ensembles d'électrodes.

13. Cellule électrochimique selon la revendication 12, dans laquelle le second ensemble d'électrodes est selon l'une quelconque des revendications 1 à 11.

14. Cellule électrochimique selon l'une ou l'autre des revendications 12 ou 13, dans laquelle la membrane échangeuse de cations comprend un fluoropolymère à base de tétrafluoroéthylène sulfoné.

15. Utilisation d'un ensemble d'électrodes selon l'une quelconque des revendications 1 à 11 ou d'une cellule électrochimique selon l'une quelconque des revendications 12 à 14 dans l'électrolyse d'un liquide, de préférence dans laquelle l'électrolyse est l'électrolyse de l'eau pour produire de l'ozone.
